# FASCICULE DE BREVET EUROPEEN

(11) **EP 0 858 501 B1**
(45) Date de publication et mention de la délivrance du brevet: **06.07.2005**
(21) Numéro de dépôt: 96937362.0
(22) Date de dépôt: 31.10.1996
(51) Int. Cl.: C12N 1/20, A23C 9/123

(54) **FERMENTS LACTIQUES, ET LEUR UTILISATION POUR L'OBTENTION DE PRODUITS HYPOCHOLESTEROLEMIANTS**
MILCHSÄUREBAKTERIEN UND IHRE ANWENDUNG ZUR HERSTELLUNG VON HYPERCHOLESTEROLEMISCHE AKTIVE PRODUKTE
LACTIC STARTERS AND USE THEREOF FOR PREPARING CHOLESTEROL-LOWERING PRODUCTS

(30) Priorité: 31.10.1995 FR 9512844
(43) Date de publication de la demande: 19.08.1998
(73) Titulaire: COMPAGNIE GERVAIS-DANONE, F-92302 Levallois Perret (FR)
(72) Inventeur: BENBADIS, Laurent, F-92160 Antony (FR); BOULEY, Christine, F-92420 Vaucresson (FR); CAYUELA, Chantal, F-75016 Paris (FR); SKROCHOWSKI, Paul, F-91480 Quincy-sous-Senart (FR)
(74) Mandataire: Vialle-Presles, Marie José
(86) Numéro de dépôt international: PCT/FR1996/001701
(87) Numéro de publication internationale: WO 1997/016529

(56) Documents cités:
- EP-A- 0 181 170
- EP-A- 0 199 535
- EP-A- 0 642 740
- EP-A- 0 671 468
- US-A- 4 590 077
- US-A- 4 797 289
- PATENT ABSTRACTS OF JAPAN vol. 012, no. 143 (C-492), 30 Avril 1988 & JP 62 258323 A (SNOW BRAND MILK PROD CO LTD), 10 Novembre 1987,

## Description

La présente Invention est relative à l'obtention de produits fermentés permettant d'abaisser le taux de cholestérol sanguin.

On sait que la composition du régime alimentaire peut influencer le métabolisme des lipides et joue en conséquence un rôle important parmi les facteurs de risque de développement des maladies cardio-vasculaires chez l'homme.

A l'heure actuelle, les stratégies diététiques de prévention de ces maladies font appel à la réduction de la consommation des matières grasses, et particulièrement des matières grasses saturées, qui sont connues pour augmenter le niveau de cholestérol sanguin.

Cependant, il est également de plus en plus préconisé d'agir qualitativement sur la prise alimentaire, en incorporant dans celle-ci des aliments dotés d'une action bénéfique sur le métabolisme lipidique, et ayant pour effet de diminuer le cholestérol sanguin et particulièrement le LDL cholestérol.

On sait par exemple que l'enrichissement de l'alimentation en fibres aurait pour effet de diminuer la lipidémie et la cholestérolémie.

On connaît également des oligosaccharides comprenant 2 à 20 monomères, et dénommés oligosaccharides non-digestibles (NDO), car ils ne sont pas digérés au niveau gastro-intestinal. Ces oligosaccharides sont fermentescibles au niveau du colon, et il a été montré que certains d'entre eux peuvent réduire le cholestérol et les triglycérides sériques chez l'animal. Parmi ces oligosaccharides, les fructo-oligosaccharides (FOS) apparaissent comme particulièrement intéressants.

Cependant, la plupart des études qui font état d'un effet hypocholestérolémiant et hypolipidémiant des FOS ont été effectuées d'une part chez l'animal, et d'autre part chez des sujets diabétiques, ou hyperlipidémiques ; cet effet n'a pas été confirmé chez les sujets sains [revue bibliographique, pages 17 et 18 de la brochure d'information « NUTRITIONAL PROPERTIES OF ACTILIGHT® » distribuée par Béghin-Mejii Industries].

Il a par ailleurs été supposé que certaines souches de bactéries peuvent exercer des effets hypocholestérolémiants [SANDERS, J. Dairy. Sci. 76, 1819-1828 (1993)]. On a montré que ces bactéries fixaient le cholestérol *in vitro* [KHEDKAR et al., Indian J. Dairy. Sci., 46, 12, 577-580, (1993)], et on a émis l'hypothèse qu'elles pourraient agir de même au niveau intestinal, détournant ainsi une partie du cholestérol apporté par l'alimentation.

La Demande de Brevet Européen 0101 209 au nom de KABUSHIKI KAISYA ADVANCE KAIHATSU KENKYUJO mentionne les propriétés hypocholestérolémiantes et hypotriglycéridémiantes d'un streptocoque, *Streptococcus faecium*.

ZACCONI et al., [Microbiologica, 15, 413-418, 1992)] ont observé, chez la souris, qu*'Enterococcus faecium* (dénomination actuelle de *Streptococcus faecium*) pouvait induire un abaissement du cholestérol sanguin ; ils ont également constaté un effet du même type, mais moindre, induit par certaines souches de *Lactobacillus acidophilus*.

Cependant, des travaux plus récents effectués chez le rat [ODA et HASHIBA, J. Nut. Sci. Vitaminol, 40, 617-621, (1994)], ou chez la souris [TANNOCK et McCONNELL, Microbial Ecology In Health And Disease, Vol. 7 : 331-334 (1994)], ne montrent aucun effet des lactobacilles sur le cholestérol sanguin.

GILLILAND et SPECK [Appl. Environ. Microbiol. 33, 15-18, (1977)] ont émis l'hypothèse d'une relation entre la capacité de certaines souches à déconjuguer les acides biliaires (taurocholate et/ou glycocholate) et à croître en présence de bile, et leurs propriétés hypocholestérolémiantes.

Ultérieurement, la même équipe GILLILAND et al. [Appl. Environ. Microbiol. 49, 377-381, (1985)] a étudié l'effet hypocholestérolémiant de différentes souches de *Lactobacillus acidophilus* chez le porc, en relation avec leur capacité *in vitro* d'assimilation du cholestérol et de croissance en présence de bile. Ces auteurs ont ainsi fait la constatation que les souches dotées de propriétés hypocholestérolémiantes étaient celles capables à la fois de croître en présence de bile et d'assimiler le cholestérol.

D'autre part, la Demande EP 0671 468 au nom de THE CALPIS FOOD INDUSTRY CO, LTD, décrit des souches de *Lactobacillus acidophilus* qui sont capables de croître en présence de bile et d'adsorber le cholestérol, mais ne déconjuguent pas les acides biliaires *in vitro.* En effet, il est considéré dans la Demande EP 0671 468 que la déconjugaison des acides biliaires constitue une caractéristique défavorable, car elle entraîne une inhibition de l'absorption des nutriments. Lorsque ces souches sont administrées à des rats, on observe chez ceux-ci une diminution du taux de cholestérol sanguin, accompagné d'un maintien du poids, qui témoigne d'une absorption normale des nutriments.

Ces résultats ont été obtenus chez des animaux, et ne peuvent pas aisément être extrapolés chez l'homme, étant donné les spécificités d'espèces qui existent au niveau métabolique.

Les études effectuées chez l'homme ont également conduit à des résultats variables :
- LIN et al., [J. Dairy. Sci, 72, 2885-2899 (1989)], relatent une absence d'effet de l'ingestion de tablettes comprenant *Lactobacillus acidophilus* et *Lactobacillus delbrueckii ssp. bulgaricus* sur la concentration de lipoprotéines et de cholestérol sanguin, bien que ces mêmes bactéries absorbent le cholestérol *in vitro.* Les auteurs attribuent au moins en partie cette divergence à la faible viabilité de ces souches dans le tractus digestif ; d'autre part, AGERBAEK et al. [EUROPEAN JOURNAL OF CLINICAL NUTRITION, 49, 346-352, (1995)], décrivent l'effet hypocholestérolémiant, sur des sujets sains, d'un lait fermenté en présence *d'Enterococcus faecium* et de deux souches de *Streptococcus thermophilus*.

KHEDKAR et al. [CULTURED DAIRY PRODUCTS JOURNAL, août 1993, pages 14-18], ont effectué une revue de divers travaux concernant l'effet de laits fermentés sur le cholestérol sanguin, et concluent que cet effet est inconstant.

Il apparaît donc que, bien l'on puisse présumer un effet hypocholestérolémiant de certains produits laitiers fermentés, l'ensemble des facteurs qui en sont responsables n'a pas été identifié avec précision.

Les Inventeurs ont maintenant constaté que indépendamment de leurs capacités à adsorber ou à assimiler le cholestérol, et à déconjuguer ou non les acides biliaires, certaines souches de *L. acidophilus* possédaient à un degré plus ou moins important, des propriétés inhibitrices de la lipase pancréatique. Or, cette enzyme hydrolyse les triglycérides pour produire des acides gras libres qui sont plus facilement absorbés au niveau de l'intestin que les triglycérides, et sont utilisés par l'organisme pour la synthèse de lipides, dont le cholestérol. Une inhibition partielle de l'activité de cette enzyme peut donc provoquer indirectement une baisse de cholestérol sanguin.

Parmi différentes souches testées sur la base non seulement de leurs propriétés à croître en présence de bile, et à assimiler le cholestérol, mais également sur celle de leurs propriétés inhibitrices de la lipase pancréatique, les Inventeurs ont sélectionné des souches sensiblement inhibitrices de cette enzyme, et possédant une bonne capacité *in vitro* à croître en présence de bile et à assimiler le cholestérol sanguin.

En outre, les Inventeurs ont constaté que ces souches, bien qu'elles soient capables de déconjuguer les acides biliaires, ne diminuent pas l'absorption des nutriments.

Ces souches constituent la base d'un nouveau produit laitier fermenté favorisant la baisse du taux de cholestérol sanguin. Elles peuvent être avantageusement associées entre elles ou à d'autres souches de bactéries lactiques.

La présente Invention a pour objet la souche de *Lactobacillus acidophilus* déposée le 6 juillet 1990, auprès de la CNCM (Collection Nationale de Cultures de Microorganismes) tenue par l'INSTITUT PASTEUR, 25 rue du Docteur Roux, à Paris, sous le numéro I-967.

En particulier, la présente Invention a pour objet un ferment lactique comprenant un mélange de la souche de *Lactobacillus acidophilus* I-967 mentionnée ci-dessus, avec au moins une autre souche de *Lactobacillus acidophilus*.

Selon un mode de réalisation préféré d'un ferment lactique conforme à la présente Invention, la souche de *Lactobacillus acidophilus* I-967 est associée avec la souche de *Lactobacillus acidophilus*, qui a été déposée le 24 Octobre 1995 auprès de la CNCM, sous le numéro I-1633.

Avantageusement, un ferment lactique conforme à la présente Invention, peut comprendre en outre au moins un lactocoque mésophile ; il s'agit de préférence de la souche de *Lactococcus lactis* ssp. *lactis*, qui a été déposée le 24 octobre 1995 auprès de la CNCM sous le numéro I-1631

Le ferment lactique conforme à l'Invention peut être associé à différentes espèces de bactéries utilisables dans la fabrication du yoghourt ou d'autres produits laitiers fermentés; de préférence, on l'associera par exemple à au moins une souche de *Streptococcus thermophilus,* et/ou au moins une souche de *Lactobacillus delbrueckii* ssp. *bulgaricus*.

Des souches de *Streptococcus thermophilus,* et de *Lactobacillus delbrueckii ssp. bulgaricus* qui peuvent être avantageusement utilisées pour la mise en oeuvre de la présente Invention sont les suivantes :
- La souche de *Lactobacillus delbrueckii ssp. bulgaricus* déposée le 30 décembre 1994 auprès de la CNCM sous le numéro I-1519 ;
- la souche de *Lactobacillus delbrueckii ssp. bulgaricus* déposée le 24 octobre 1995 auprès de la CNCM sous le numéro I-1632 ;
- la souche de *Streptococcus thermophilus* déposée le 24 octobre 1995 auprès de la CNCM sous le numéro I-1630.

La présente Invention a également pour objet un procédé de préparation d'un produit fermenté, lequel procédé est caractérisé en ce qu'il comprend la mise en oeuvre d'un ferment lactique conforme à l'Invention éventuellement associé à au moins une souche de *Streptococcus thermophilus,* et/ou au moins une souche de *Lactobacillus delbrueckii ssp. bulgaricus*.

La présente Invention a également pour objet les produits fermentés, en particulier des produits laitiers fermentés, susceptibles d'être obtenus par un procédé conforme à l'Invention, tel que défini ci-dessus.

Selon un mode de réalisation préféré d'un produit fermenté conforme à l'Invention, il contient après fermentation au moins 1×10⁵ UFC (=Unités Formant Colonie) par ml de chacune des espèces bactériennes qui constituent le ferment.

Une efficacité optimale d'un produit fermenté conforme à l'Invention a été observée pour une concentration minimale d'environ 1×10⁶ à 1×10⁷ UFC par ml de chaque espèce bactérienne du ferment.

Selon un autre mode de réalisation préféré d'un produit laitier fermenté conforme à l'Invention, il comprend en outre des fructo-oligosaccharides (FOS). Selon une disposition préférée de ce mode de réalisation, ledit produit laitier fermenté comprend entre 0,1 et 20 g pour 100 ml, et de préférence entre 1 et 10 g pour 100 ml de fructo-oligosaccharides.

Les fructo-oligosaccharides sont des oligomères glucidiques, qui peuvent être obtenus par l'action d'une transfructosidase sur le saccharose.

Ils sont constitués de fructose(F), et de glucose(G), et répondent à la formule générale G(Fn), dans laquelle n, qui est supérieur ou égal à 2 représente le nombre de molécules de fructose.

Pour la mise en oeuvre de la présente Invention, on utilisera avantageusement des fructo-oligosaccharides dans lesquels n est inférieur ou égal à 8, et de préférence inférieur ou égal à 4.

Selon encore un autre mode de réalisation préféré, un produit laitier fermenté conforme à l'Invention comprend entre 0,05 et 20 g pour 100 ml, et de préférence entre 0,1 et 10 g pour 100 ml de matières grasses d'origine végétale et/ou entre 0,05 et 15 g pour 100 ml, et de préférence entre 0,1 et 5 g pour 100 ml de matières grasses d'origine animale.

Les matières grasses d'origine végétale peuvent être apportées par l'utilisation d'au moins une huile végétale telle que l'huile de colza, l'huile de soja, les huiles d'olive, de germe de blé, de mais, de tournesol, de pépins de raisin, de pépin de cassis, etc....; les matières grasses d'origine animale peuvent être des matières grasses laitières, apportées par exemple par ajout de crème, et/ou des huiles de poisson.

Ces matières grasses peuvent également être enrichies en vitamines liposolubles, en particulier en vitamine E, de manière à ce que le produit fermenté prêt à la consommation contienne entre 0,03mg et 10mg pour 100ml.

La consommation régulière de produits laitiers fermentés conforme à l'Invention induit une baisse significative du taux de cholestérol sanguin, et en particulier du LDL cholestérol.

La présente Invention englobe également l'utilisation de souches bactériennes, de ferments lactiques, ou de produits fermentés conformes à l'Invention pour l'obtention de produits induisant une baisse du taux de cholestérol sanguin.

La présente Invention sera mieux comprise à l'aide du complément de description qui va suivre, qui se réfère à des exemples de préparation et d'utilisation de produits fermentés conformes à l'Invention.

Il doit être bien entendu toutefois que ces exemples sont donnés uniquement à titre d'illustration de l'objet de l'Invention dont ils ne constituent en aucune manière une limitation.

### EXEMPLE 1 - OBTENTION D'UN PRODUIT LAITIER FERMENTE CONFORME A L'INVENTION

On prépare un mélange, dont la composition (en % p/p) est donnée dans le Tableau I ci-dessous :

**TABLEAU 1**

| INGREDIENT | QUANTITE (%) |
|---|---|
| Lait écrémé | 90 |
| Crème (40%MG) | 1,3 |
| Poudre de lait 0%MG | 5,5 |
| Huile de soja ^{a} | 0,25 |
| Huile de colza ^{a} | 0,25 |
| ACTILIGHT®950P ^{b} (Fructo-oligosaccharides) | 2,50 |
| Stabilisant (Gélatine) | 0,20 |

| | |
|---|---|
| ^{a} Les huiles de soja et de colza sont enrichies en vitamine E et apportent au total 0,35 mg de vitamine E (exprimée sous forme de α-dl tocophérol) pour 100g de mélange. | |
| ^{b} ACTILIGHT®950P: Préparation à plus de 90% de fructo-oligosaccharides, commercialisée par BEGHIN-MEIJII INDUSTRIES. | |

Les ingrédients sont mélangés pendant 1 heure sous agitation, de manière à obtenir une bonne réhydratation de la poudre de lait, et un mélange homogène.

Le mélange est ensuite chauffé à 75°C préalablement à l'homogénéisation. Après homogénéisation à 200 bars, le mélange est pasteurisé, et refroidi avant d'être ensemencé à 38°C avec des cultures actives des différentes souches.

La fermentation est effectuée dans une étuve régulée à 38±1°C, jusqu'à un pH de 4,75±0,15. L'arrêt de la fermentation est effectué par refroidissement de la préparation jusqu'à 20°C environ. Le produit est ensuite conditionné et réfrigéré à 4°C.

Les concentrations en début de fermentation (concentration initiale) et en fin de fermentation (concentration finale) sont indiquées (en UFC par ml) dans le Tableau II ci-dessous :

**TABLEAU II**

| SOUCHE | CONCENTRATION | |
|---|---|---|
| | INITIALE | FINALE |
| *Lactobacillus acidophilus* (I-1633)+(I-967) | 3,3 × 10⁶ | 1,2 × 10⁸ |
| *Lactobacillus* totaux | 4,8 × 10⁶ | 3,7 × 10⁸ |
| *Lactococcus lactis* ssp *lactis* (I-1631) | 1,4 × 10⁶ | 6,7 × 10⁷ |
| *Lactobacillus delbrueckii* ssp *bulgaricus* (I-1519)+(I-1632) | 1,5 × 10⁶ | 2,5 × 10⁸ |
| *Streptococcus thermophilus* (I-1630) | 1,5 × 10⁵ | 1, 6 × 10⁹ |

Les méthodes utilisées pour le dénombrement des bactéries sont les suivantes :

*Lactobacillus* totaux : ensemencement en masse sur gélose MRS acide (pH =5,4) ; incubation 72 heures à 37°C en anaérobiose.

*Lactobacillus acidophilus* : ensemencement en masse sur gélose MRS acide OXGALL (0,2%); incubation 72 heures à 37°C en anaérobiose.

*Lactobacillus delbrueckii* ssp *bulgaricus* : la population est évaluée en calculant la différence : (Lactobacillus totaux) - (Lactobacillus *acidophilus*).

*Streptococcus thermophilus* : ensemencement en masse sur gélose M17 ; incubation 48 heures à 44°C.

*Lactococcus lactis* : ensemencement en masse sur gélose M17 ; incubation 5 jours à 17°C.

### EXEMPLE 2 - ACTION D'UN PRODUIT LAITIER FERMENTE CONFORME A L'INVENTION, SUR LE CHOLESTEROL SANGUIN CHEZ L'HOMME.

L'effet hypocholestérolémiant d'un produit fermenté conforme à l'Invention a été étudié en utilisant comme témoin un yoghourt, comprenant 1% de graisse animale, et fermenté avec uniquement les ferments du yoghourt.

Le produit fermenté conforme à l'Invention a été obtenu selon le procédé décrit à l'exemple 1, et comprend 0,5% de graisse végétale, 0,5% de graisse animale, et 2,5% de fructo-oligosaccharides.

L'étude de l'effet hypocholestérolémiant a été effectuée, dans un essai randomisé croisé en double-aveugle, chez 30 sujets mâles en bonne santé, âgés de 30 à 65 ans. Ces sujets ont été répartis en deux groupes ; chacun de ces deux groupes comprend 60% d'homozygotes et 40% d'hétérozygotes pour l'apolipoprotéine E3 ; les sujets homozygotes pour l'apolipoprotéine E3, qui est impliquée dans le métabolisme des lipides, sont supposés être plus sensibles à l'influence de la composition en matières grasses du régime alimentaire sur ce métabolisme.

Un premier groupe de 15 sujets sains a reçu pendant 3 semaines un produit fermenté conforme à l'Invention ; après une interruption d'une semaine, les mêmes sujets ont reçu pendant 3 semaines le yoghourt de contrôle ; parallèlement, le second groupe de sujets sains a reçu pendant les 3 premières semaines le yoghourt de contrôle, puis, après une interruption d'une semaine, a reçu pendant 3 semaines le produit fermenté conforme à l'Invention.

Pendant chacune des périodes de traitement, chaque sujet a consommé, en plus de son régime habituel 3 × 125 g de produit réparti en 3 prises quotidiennes, dans le cadre des repas habituels (petit déjeuner, déjeuner et dîner).

Le cholestérol sanguin a été dosé chez les sujets en début du traitement, à la fin de la première période, et à la fin de la seconde période ; les résultats, représentant la moyenne des dosages effectués après la consommation de yoghourt contrôle, et après la consommation du produit fermenté conforme à l'Invention, sont résumés dans le Tableau III ci-dessous :

**TABLEAU III**

| | TRAITEMENT | |
|---|---|---|
| | CONTROLE | TEST |
| CHOLESTEROL TOTAL (mmol/l) | 5,41 | 5,17 |
| CHOLESTEROL LDL (mmol/l) | 3,52 | 3,33 |
| CHOLESTEROL HDL (mmol/l) | 1,23 | 1,21 |
| RAPPORT LDL CHOLESTEROL/HDL CHOLESTEROL | 3,02 | 2,86 |

L'analyse statistique des résultats à la fin de la première et à la fin de la seconde période, et de la moyenne des rapports (LDL CHOLESTEROL/HDL CHOLESTEROL) obtenus pour chacun des individus à la fin des deux périodes a permis de mettre en évidence une diminution significative du cholestérol sérique total (-4,3% ; p<0,001) ainsi que du cholestérol sérique LDL (-5,4% ; p<0,005) et du rapport LDL CHOLESTEROL/HDL CHOLESTEROL (-5,4% ; p<0,05), chez les sujets ayant absorbé le produit fermenté de l'Invention, par rapport à ceux qui ont consommé le yoghourt contrôle.

Ces résultats, qui ont été observés chez des sujets n'ayant fait l'objet d'aucune restriction alimentaire, sont du même ordre de grandeur, en particulier en ce qui concerne la diminution du rapport LDL/HDL, que ceux qui ont été observés antérieurement chez des sujets soumis à un régime restrictif (qui a entraîné une diminution d'environ 7% du rapport LDL CHOLESTEROL /HDL CHOLESTEROL) [VAN DOKKUM et al., Eur. J. Clin. Nutr., 45, 431-439, (1991)].

En revanche, aucune différence significative n'a été observée en ce qui concerne le cholestérol HDL ; il en est de même pour le niveau de triglycérides, et le niveau de glucose sanguin (résultats non montrés).

D'autre part, les sujets homozygotes pour l'apolipoprotéine E3 n'ont pas réagi différemment des hétérozygotes.

### EXEMPLE 3 - CARACTERISATION DES SOUCHES DE LACTOBACILLUS ACIDOPHILUS UTILISEES POUR L'OBTENTION DE PRODUITS LAITIERS CONFORMES A L'INVENTION.

### 1) Croissance et métabolisme :

Les propriétés des souches I-967 et I-1633 ont été testées sur galerie API 50 CH.

Les résultats sont illustrés par le tableau IV ci-dessous :

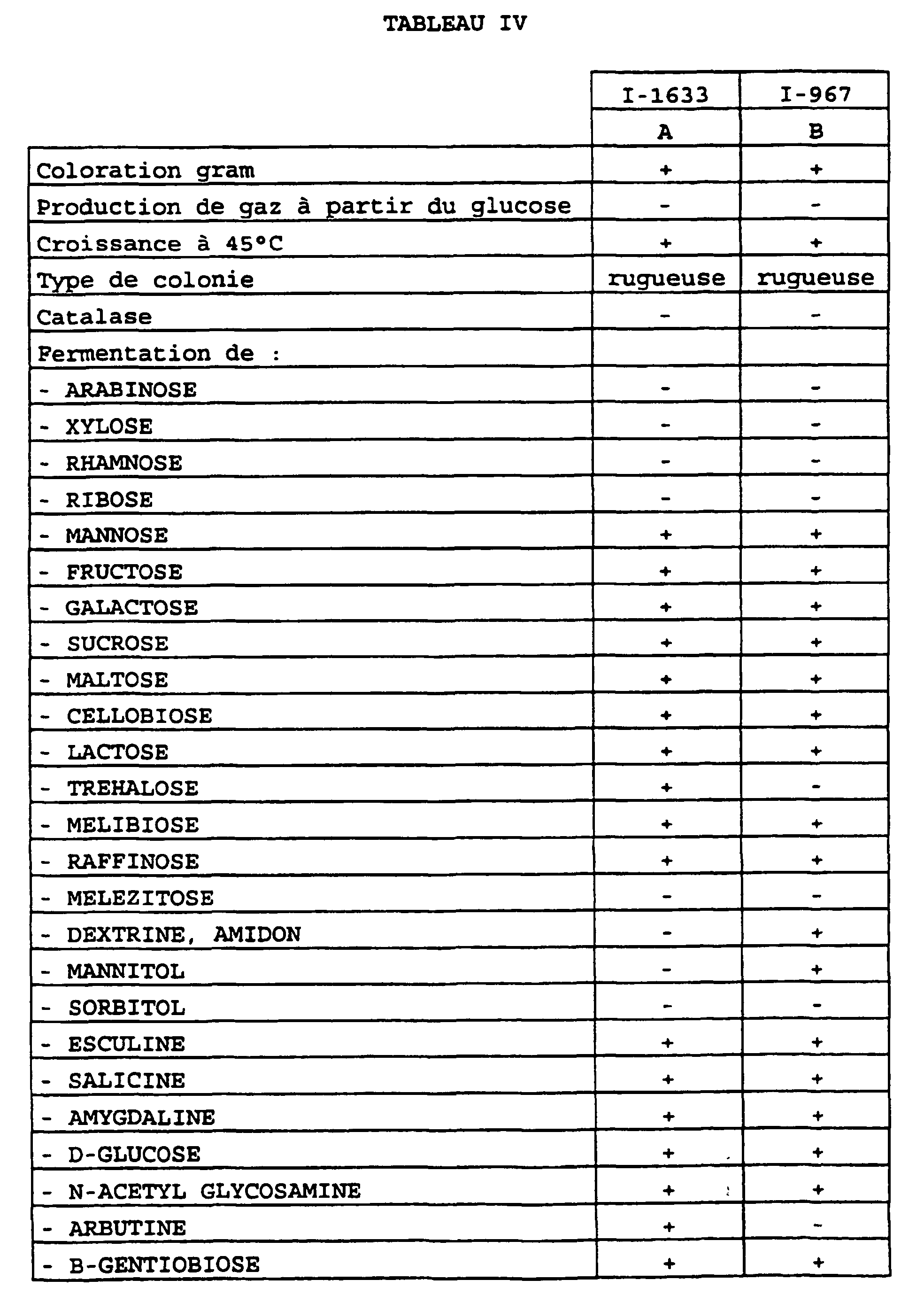

### 2) Déconjugaison des acides biliaires

La capacité des souches à déconjuguer l'acide taurodeoxycholique (TDCA) et l'acide glycodeoxycholique (GDCA), a été évaluée en utilisant le protocole décrit par DASHKEVICZ et FEIGHNEIR, [Appl. and Environ. Microbiol., 55, 1, p. 11-16 (1989)]. Dans le cas du GDCA, ce protocole a été modifié en utilisant 0,15% de GDCA à la place des 0,5% décrits dans la publication.

Les souches I-967 et I-1633 déconjuguent le TDCA. Le GDCA est également déconjugué, mais de manière moins importante et moins reproductible.

### 3) Inhibition in vitro de la lipase pancréatique

Les souches de *Lactobacillus acidophilus*, I-967 et I-1633, ainsi que les souches témoins 1 et 2 ont été mises en culture.

Après la culture, les cellules sont collectées et dénombrées. Elles sont lavées et mises en suspension dans du sérum physiologique, à des concentrations de l'ordre de 10⁶-10⁸ cellules/ml et utilisées dans les heures suivantes.

Le dosage de la lipase pancréatique est réalisé comme suit :
Le mélange réactionnel contenant 14,5 ml de NacL 150 mM à pH 7,5 et 0,5 ml de tributyrine est maintenu sous agitation mécanique à une température régulée à 25°C. Les acides gras libérés sont mesurés par titration à la soude à l'aide d'un titreur pH stat.

On utilise une lipase pancréatique purifiée de porc (Boehringer), à raison de 10 à 20 unités enzymatiques par dosage (une unité = une µmol d'acide gras libérée/min), en présence d'une colipase pancréatique purifiée de porc (Boehringer), en quantité saturante.

On mesure la cinétique de la réaction jusqu'à obtention d'un enregistrement linéaire.

Le dosage est effectué dans deux conditions différentes :
- en présence de sels biliaires purifiés, à la concentration finale de 8 mM
- en présence de bile de porc dialysée, avec une concentration finale de sels biliaires totaux de 8 mM.

Le pouvoir inhibiteur des souches de *Lactobacillus acidophilus* vis à vis de la lipase pancréatique est déterminé par ajout de quantités croissantes de cellules dans le milieu de réaction. Les mesures sont effectuées en double.

La courbe d'inhibition est tracée et l'activité inhibitrice est calculée selon la définition suivante : une unité inhibitrice est la quantité de cellules qui réduit de 50% l'activité de la lipase pancréatique dans les conditions du dosage.

### RESULTATS ET DISCUSSION

L'addition de cellules de différentes souches de *Lactobacillus acidophilus* sur l'activité enzymatique de la lipase pancréatique est illustrée par le tableau V ci-dessous :

**TABLEAU V**

| Souches | Quantité de cellules (x10⁸) pour 50% d'inhibition | |
|---|---|---|
| | Avec sels biliaires | Avec bile |
| Témoin 1 | 80,00 | 68,20 |
| I-967 | 24,50 | 23,60 |
| I-1633 | 10,12 | 10,87 |
| Témoin 2 | 74,10 | 48,20 |

Les souches I-967 et I-1633, pour lesquelles une unité inhibitrice correspond à une quantité de cellules inférieure à 50x10⁸ sont définies comme sensiblement inhibitrices, en référence aux souches témoins 1 et 2, pour lesquelles une unité inhibitrice correspond à une quantité de cellules supérieure à 50x10⁸.

Référence du dossier du déposant ou du mandataire | M[?] [?]d191/132 | Demande internationale n

INDICATIONS RELATIVES A UN MICRO-ORGANISME DEPOSE

(règle 13bis du PCT)

A. Les indications ont trait au micro-organisme visé dans la description
page 5 . ligne 18

B. IDENTIFICATION DU DEPOT | D'autres dépôts font l'objet d'une feuille supplementaire | X

Nom de l'institution de dépôt
CNCM (COLLECTION NATIONALE DE CULTURES DE MICROORGANISMES)

Adresse de l'institution de dépôt (y compris le code postal et le pays)
INSTITUT PASTEUR
25, rue du Docteur Roux
75015 PARIS
FRANCE

Date du depôt 6 JUILLET 1990 | n° d'ordre I-967

C. INDICATIONS SUPPLEMENTAIRES (le cas echeant ) | Une feuille supplementaire est jointe pour la suite de ces renseignements | [ ]

D. ETATS DESIGNES POUR LESQUELS LES INDICATIONS SONT DONNEES
(si les indications ne sont pas donnees pour tous les Etats designes)

E. INDICATIONS FOURNIES SEPAREMENT (le cas echeant)

Les indications enumerees ci-apres seront fournies ultérieurement au Bureau international (specifier la nature générale des indications p. ex., "n° d'ordre du dépôt")

Réservé a l'office récepteur | Réservé au Bureau international
[X] Cette feuille a été reçue en même temps que la demande internationale | [ ] Cette feuille est parvenue au Bureau international le :
Fonctionnaire autorisé | Fonctionnaire autorisé
J. LE PICAUD |

Référence du dossier du déposant ou du mandataire | M nd191/132 | Demande internationale :

INDICATIONS RELATIVES A UN MICRO-ORGANISME DEPOSE

(règle 13bis du PCT)

A. Les indications ont trait au micro-organisme visé dans la description
page 5 , ligne 29 .

B. IDENTIFICATION DU DEPOT | D'autres dépôts font l'objet d'une feuille supplémentaire | X

Nom de l'institution de dépôt
CNCM (COLLECTION NATIONALE DE CULTURES DE MICROORGANISMES)

Adresse de l'institution de dépôt (y compris le code postal et le pays)
INSTITUT PASTEUR
25, rue du Docteur Roux
75015 PARIS
FRANCE

Date du dépôt 24 OCTOBRE 1995 | n° d'ordre I-1633

C. INDICATIONS SUPPLEMENTAIRES (le cas échéant) | Une feuille supplémentaire est jointe pour la suite de ces renseignements | [ ]

D. ETATS DESIGNES POUR LESQUELS LES INDICATIONS SONT DONNEES
(si les indications ne sont pas données pour tous les Etats désignés)

E. INDICATIONS FOURNIES SEPAREMENT (le cas échéant)

Les indications énumérées ci-après seront fournies ultérieurement au Bureau international (spécifier la nature générale des indications p. ex., "n° d'ordre du dépôt")

Réservé à l'office récepteur | Réservé au Bureau international

[X] Cette feuille a été reçue en même temps que la demande internationale | [ ] Cette feuille est parvenue au Bureau international le :

Fonctionnaire autorisé | Fonctionnaire autorisé

J. LE PICAUD

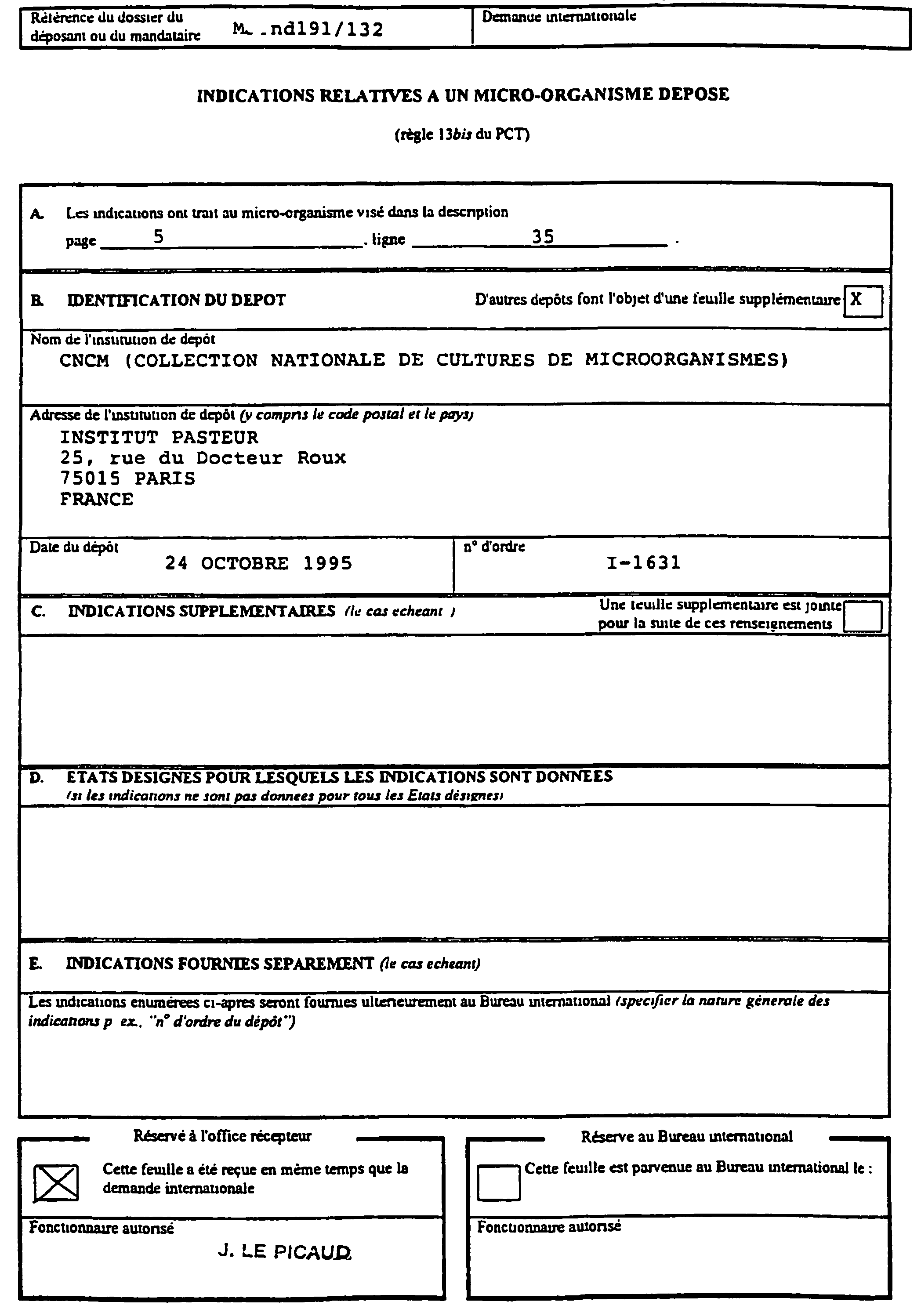
Référence du dossier du déposant ou du mandataire | M[?] .nd191/132 | Demande internationale

INDICATIONS RELATIVES A UN MICRO-ORGANISME DEPOSE

(règle 13bis du PCT)

A. Les indications ont trait au micro-organisme visé dans la description
page 5 . ligne 35 .

B. IDENTIFICATION DU DEPOT | D'autres dépôts font l'objet d'une feuille supplémentaire X

Nom de l'institution de dépôt
CNCM (COLLECTION NATIONALE DE CULTURES DE MICROORGANISMES)

Adresse de l'institution de dépôt (y compris le code postal et le pays)
INSTITUT PASTEUR
25, rue du Docteur Roux
75015 PARIS
FRANCE

Date du dépôt | n° d'ordre
24 OCTOBRE 1995 | I-1631

C. INDICATIONS SUPPLEMENTAIRES (le cas echeant ) | Une feuille supplementaire est jointe pour la suite de ces renseignements

D. ETATS DESIGNES POUR LESQUELS LES INDICATIONS SONT DONNEES
(si les indications ne sont pas donnees pour tous les Etats désignés)

E. INDICATIONS FOURNIES SEPAREMENT (le cas echeant)

Les indications enumérees ci-apres seront fournies ulterieurement au Bureau international (specifier la nature generale des indications p ex., "n° d'ordre du dépôt")

Réservé à l'office récepteur | Réservé au Bureau international

 Cette feuille a été reçue en même temps que la demande internationale | Cette feuille est parvenue au Bureau international le :

Fonctionnaire autorisé | Fonctionnaire autorisé
J. LE PICAUD

Reference du dossier du déposant ou du mandataire | [?]. [?]nd191/132 | Demande internationale

INDICATIONS RELATIVES A UN MICRO-ORGANISME DEPOSE

(règle 13bis du PCT)

A. Les indications ont trait au micro-organisme visé dans la description
page 6 , ligne 14 .

B. IDENTIFICATION DU DEPOT | D'autres dépôts font l'objet d'une feuille supplémentaire | X

Nom de l'institution de dépôt
CNCM (COLLECTION NATIONALE DE CULTURES DE MICROORGANISMES)

Adresse de l'institution de dépôt (y compris le code postal et le pays)
INSTITUT PASTEUR
25, rue du Docteur Roux
75015 PARIS
FRANCE

Date du dépôt | n° d'ordre
30 DECEMBRE 1994 | I-1519

C. INDICATIONS SUPPLEMENTAIRES (le cas échéant) | Une feuille supplementaire est jointe pour la suite de ces renseignements | [ ]

D. ETATS DESIGNES POUR LESQUELS LES INDICATIONS SONT DONNEES
(si les indications ne sont pas donnees pour tous les Etats désignés)

E. INDICATIONS FOURNIES SEPAREMENT (le cas echéant)

Les indications énumérees ci-apres seront fournies ulterieurement au Bureau international (specifier la nature generale des indications p. ex., "n° d'ordre du dépôt")

Réservé à l'office récepteur | Réserve au Bureau international
[X] Cette feuille a éte reçue en même temps que la demande internationale | [ ] Cette feuille est parvenue au Bureau international le :
Fonctionnaire autorisé | Fonctionnaire autorise
J. LE [?][?][?][?][?] |

Reference du dossier du déposant ou du mandataire | M. nd191/132 | Demande internationale ·

INDICATIONS RELATIVES A UN MICRO-ORGANISME DEPOSE

(règle 13bis du PCT)

A. Les indications ont trait au micro-organisme visé dans la description
page 6 , ligne 17 .

B. IDENTIFICATION DU DEPOT | D'autres dépôts font l'objet d'une feuille supplémentaire [X]

Nom de l'institution de dépôt
CNCM (COLLECTION NATIONALE DE CULTURES DE MICROORGANISMES)

Adresse de l'institution de dépôt (y compris le code postal et le pays)
INSTITUT PASTEUR
25, rue du Docteur Roux
75015 PARIS
FRANCE

Date du dépôt 24 OCTOBRE 1995 | n° d'ordre I-1632

C. INDICATIONS SUPPLEMENTAIRES (le cas échéant ) | Une feuille supplémentaire est jointe pour la suite de ces renseignements [ ]

D. ETATS DESIGNES POUR LESQUELS LES INDICATIONS SONT DONNEES
(si les indications ne sont pas données pour tous les Etats désignés)

E. INDICATIONS FOURNIES SEPAREMENT (le cas échéant)

Les indications énumérées ci-après seront fournies ultérieurement au Bureau international (spécifier la nature générale des indications p. ex., "n° d'ordre du dépôt")

Réservé à l'office récepteur | Réservé au Bureau international
[X] Cette feuille a été reçue en même temps que la demande internationale | [ ] Cette feuille est parvenue au Bureau international le :
Fonctionnaire autorisé [?] | Fonctionnaire autorisé

Reference du dossier du déposant ou du mandataire | M[?] nd191/132 | Demande internationale :

INDICATIONS RELATIVES A UN MICRO-ORGANISME DEPOSE

(règle 13bis du PCT)

A. Les indications ont trait au micro-organisme visé dans la description
page 20 , ligne 6 .

B. IDENTIFICATION DU DEPOT | D'autres dépôts font l'objet d'une feuille supplémentaire [ ]

Nom de l'institution de dépôt
CNCM (COLLECTION NATIONALE DE CULTURES DE MICROORGANISMES)

Adresse de l'institution de dépôt (y compris le code postal et le pays)
INSTITUT PASTEUR
25, rue du Docteur Roux
75015 PARIS
FRANCE

Date du dépôt | n° d'ordre
24 OCTOBRE 1995 | I-1630

C. INDICATIONS SUPPLEMENTAIRES (le cas échéant ) | Une feuille supplementaire est jointe pour la suite de ces renseignements [ ]

D. ETATS DESIGNES POUR LESQUELS LES INDICATIONS SONT DONNEES
(si les indications ne sont pas donnees pour tous les Etats designes)

E. INDICATIONS FOURNIES SEPAREMENT (le cas échéant)

Les indications enumerées ci-apres seront fournies ultérieurement au Bureau international (specifier la nature generale des indications p. ex., "n° d'ordre du dépôt")

Réserve à l'office récepteur | Réserve au Bureau international
[X] Cette feuille a été reçue en même temps que la demande internationale | [ ] Cette feuille est parvenue au Bureau international le :
Fonctionnaire autorisé | Fonctionnaire autorise
[?]. LE [?][?]CAUD |

## Revendications

1. Souche de *Lactobacillus acidophilus* choisie parmi :
- la souche déposée le 6 juillet 1990, auprès de la CNCM sous le numéro I-967.
- la souche déposée le 24 octobre 1995, auprès de la CNCM sous le numéro I-1633.

2. Ferment lactique comprenant un mélange de la souche de *Lactobacillus acidophilus* I-967 selon la revendication 1 avec au moins une autre souche de *Lactobacillus acidophilus*.

3. Ferment lactique selon la revendication 2, **caractérisé en ce qu'**il comprend un mélange des souches de *Lactobacillus acidophilus* I-967 et I-1633 selon la revendication 1.

4. Ferment lactique selon une quelconque des revendications 2 ou 3, **caractérisé en ce qu'**il comprend en outre au moins un lactocoque mésophile.

5. Ferment lactique selon la revendication 4 **caractérisé en ce que** ledit lactocoque mésophile est constitué par la souche de *Lactococcus lactis* ssp *lactis*, qui a été déposée le 24 octobre 1995 auprès de la CNCM sous le numéro I-1631

6. Procédé de préparation d'un produit fermenté, **caractérisé en ce qu'**il comprend la mise en oeuvre d'une souche selon la revendication 1 ou d'un ferment lactique selon une quelconque des revendications 2 à 5, éventuellement associé à au moins une souche de *Streptococcus thermophilus* et/ou au moins une souche de *Lactobacillus delbrueckii ssp. bulgaricus*.

7. Procédé selon la revendication 6 **caractérisé en ce que** ledit ferment lactique est associé à au moins une souche du groupe constitué par :
- la souche de *Lactobacillus delbrueckii ssp. bulgaricus* déposée le 30 décembre 1994 auprès de la CNCM sous le numéro I-1519 ;
- la souche de *Lactobacillus delbrueckii ssp. bulgaricus* déposée le 24 octobre 1995 auprès de la CNCM sous le numéro I-1632
- la souche de *Streptococcus thermophilus* déposée le 24 octobre 1995 auprès de la CNCM sous le numéro I-1630.

8. Produit fermenté, **caractérisé en ce qu'**il est susceptible d'être obtenu par un procédé selon une quelconque des revendications 6 ou 7 et contient les bactéries d'un ferment lactique selon une quelconque des revendications 2 à 5.

9. Produit fermenté selon la revendication 8, **caractérisé en ce qu'**il contient, après fermentation, au moins 1×10⁵ UFC par ml, de chacune des bactéries qui constituent le ferment selon une quelconque des revendications 2 à 5.

10. Produit fermenté selon une quelconque des revendications 8 ou 9, **caractérisé en ce qu'**il s'agit d'un produit laitier, et **en ce qu'**il comprend en outre des fructo-oligosaccharides (FOS).

11. Produit laitier fermenté selon la revendication 10 **caractérisé en ce qu'**il comprend 0,1 à 20 g de fructo-oligosaccharides pour 100 ml.

12. Produit laitier fermenté selon une quelconque des revendications 10 ou 11 **caractérisé en ce qu'**il comprend des fructo-oligosaccharides qui répondent à la formule générale G(Fn), dans laquelle G représente le glucose, F représente le fructose, et n représente le nombre de molécules de fructose qui est supérieur ou égal à 2, et inférieur ou égal à 8.

13. Produit laitier fermenté selon une quelconque des revendications 8 à 12, **caractérisé en ce qu'**il comprend 0,05 à 20 g pour 100 ml de matières grasses d'origine végétale et/ou 0,05 à 15 g pour 100 ml de matières grasses d'origine animale.

14. Utilisation d'une souche bactérienne selon la revendication 1, pour l'obtention de produits induisant une baisse du taux de cholestérol sanguin.

15. Utilisation d'un ferment lactique selon une quelconque des revendications 2 à 5, pour l'obtention de produits induisant une baisse du taux de cholestérol sanguin.

16. Utilisation d'un produit fermenté selon une quelconque des revendications 8 à 13, pour l'obtention de produits induisant une baisse du taux de cholestérol sanguin.

## Patentansprüche

1. *Lactobacillus acidophilus*-Stamm, ausgewahlt aus:
- dem Stamm, der am 6. Juli 1990 gemäß CNCM unter der Nummer I-967 hinterlegt wurde;
- dem Stamm, der am 24. Oktober 1995 gemaß CNCM unter der Nummer 1-1633 hinterlegt wurde.

2. Milchferment, umfassend ein Gemisch des Stamms *Lactobacillus acidophilus* I-967 gemäß Anspruch 1 mit mindestens einem anderen *Lactobacillus acidophilus*-Stamm.

3. Milchferment nach Anspruch 2, **dadurch gekennzeichnet, dass** es ein Gemisch der Stämme *Lactobacillus acidophilus* I-967 und I-1633 nach Anspruch 1 umfasst.

4. Milchferment nach Anspruch 2 oder Anspruch 3, **dadurch gekennzeichnet, dass** es außerdem mindestens eine mesophile Laktokokke umfasst.

5. Milchferment nach Anspruch 4, **dadurch gekennzeichnet, dass** die mesophile Laktokokke durch den Stamm *Lactococcus lactis ssp. lactis* gebildet wird, der am 24. Oktober 1995 gemaß CNCM unter der Nummer I-1631 hinterlegt wurde.

6. Verfahren zur Herstellung eines fermentierten Produktes, **dadurch gekennzeichnet, dass** es die Verwendung eines Stamms nach Anspruch 1 oder eines Milchferments nach einem der Anspruche 2 bis 5, gegebenenfalls assoziiert mit mindestens einem *Streptococcus thermophilus*-Stamm und/oder mindestens einem *Lactobacillus delbrueckii ssp. bulgaricus*-Stamm umfasst.

7. Verfahren nach Anspruch 6, **dadurch gekennzeichnet, dass** das Milchferment mit mindestens einem Stamm der Gruppe, bestehend aus:
- dem *Lactobacillus delbrueckii ssp. bulgaricus*-Stamm, der am 30. Dezember 1994 gemäß CNCM unter der Nummer 1-1519 hinterlegt wurde;
- dem *Lactobacillus delbrueckii ssp. bulgaricus*-Stamm, der am 24. Oktober 1995 gemäß CNCM unter der Nummer 1-1632 hinterlegt wurde;
- dem *Streptococcus thermophilus*-Stamm, der am 24. Oktober 1995 gemäß CNCM unter der Nummer 1-1630 hinterlegt wurde, assoziiert ist.

8. Fermentiertes Produkt, **dadurch gekennzeichnet, dass** es durch ein Verfahren nach Anspruch 6 oder 7 erhalten werden kann und die Bakterien eines Milchferments nach einem der Anspruche 2 bis 5 enthält.

9. Fermentiertes Produkt nach Anspruch 8, **dadurch gekennzeichnet, dass** es nach Fermentation mindestens 1x10⁵ kbE pro ml von jedem der Bakterien, die das Ferment nach einem der Ansprüche 2 bis 5 bilden, enthält.

10. Fermentiertes Produkt nach Anspruch 8 oder 9, **dadurch gekennzeichnet, dass** es sich um ein Milchprodukt bzw. Molkereiprodukt handelt und dass es außerdem Fructo-Oligosaccharide (FOS) umfasst.

11. Fermentiertes Milchprodukt nach Anspruch 10, **dadurch gekennzeichnet, dass** es 0,1 bis 20 g Fructo-Oligosaccharide pro 100 ml umfasst.

12. Fermentiertes Milchprodukt nach Anspruch 10 oder 11, **dadurch gekennzeichnet, dass** es Fructo-Oligosaccharide umfasst, die der allgemeinen Formel G(Fn) entsprechen, worin G fur Glucose steht, F fur Fructose steht und n die Anzahl der Fructose-Moleküle darstellt, die gleich 2 oder daruber und gleich 8 oder darunter ist.

13. Fermentiertes Milchprodukt nach einem der Ansprüche 8 bis 12, **dadurch gekennzeichnet, dass** es pro 100 ml 0,05 bis 20 g Fett pflanzlichen Ursprungs und/oder pro 100 ml 0,05 bis 15 g Fett tierischen Ursprungs umfasst.

14. Verwendung eines Bakterienstamms nach Anspruch 1 zum Erhalt von Produkten, die eine Senkung des Blutcholesterinspiegels induzieren.

15. Verwendung eines Milchferments nach einem der Ansprüche 2 bis 5 zum Erhalt von Produkten, die eine Senkung des Blutcholesterinspiegels induzieren.

16. Verwendung eines fermentierten Produktes nach einem der Ansprüche 8 bis 13 zum Erhalt von Produkten, die eine Senkung des Blutcholesterinspiegels induzieren.

## Claims

1. Strain of *Lactobacillus acidophilus* chosen from amongst:
- the strain filed on 6 July 1990 with the CNCM under the number 1-967,
- the strain filed on 24 October 1995 with the CNCM under the number I-1633.

2. Lactic ferment comprising a mixture of the *Lactobacillus acidophilus* 1-967 strain according to claim 1 with at least one other strain of *Lactobacillus acidophilus*.

3. Lactic ferment according to claim 2, **characterised in that** it comprises a mixture cf *Lactobacillus acidophilus* 1-967 and I-1633 strains according to claim 1.

4. Lactic ferment according to either one of claims 2 or 3, **characterised in that** it also comprises at least one mesophilic Lactococcus.

5. Lactic ferment according to claim 4, **characterised in that** the said mesophilic Lactococcus consists of the *Lactococcus lactis ssp lactis* strain which was filed on 24 October 1995 with the CNCM under the number I-1631.

6. Method of preparing a fermented product, **characterised in that** it comprises the use of a strain according to claim 1 or a lactic ferment according to any one of claims 2 to 5, possibly associated with at least one strain of *Streptococcus thermophilus* and/or at least one strain of *Lactobacillus delbrueckii ssp. bulgaricus*.

7. Method according to claim 6, **characterised in that** the said lactic ferment is associated with at least one strain in the group consisting of :
- the *Lactobacillus delbrueckii ssp. bulgaricus* strain filed on 30 December 1994 with the CNCM under the number I-1519;
- the *Lactobacillus delbrueckii ssp bulgaricus* strain filed on 24 October 1995 with the CNCM under the number I-1632;
- the *Streptococcus thermophilus* strain filed on 24 October 1995 with the CNCM under the number I-1630.

8. Fermented product, **characterised in that** it is obtainable by a method according to either one of claims 6 or 7 and contains the bacteria of a lactic ferment according to any one of claims 2 to 5.

9. Fermented product according to claim 8, **characterised in that** it contains, after fermentation, at least 1 x 10⁵ UFC per ml of each of the bacteria which constitute the ferment according to any one of claims 2 to 5.

10. Fermented product according to either one of claims 8 or 9, **characterised in that** it is a milk product, and **in that** it also comprises fructo-oligosaccharides (FOS).

11. Fermented milk product according to claim 10, **characterised in that** it comprises 0.1 to 20 g of fructo-oligosaccharides per 100 ml.

12. Fermented milk product according to either one of claims 10 or 11, **characterised in that** it comprises fructo-oligosaccharides which comply with the general formula G(Fn), in which G represents glucose, F represents fructose and n represents the number of molecules of fructose, which is greater than or equal to 2 and less than or equal to 8.

13. Fermented milk product according to any one of claims 8 to 12, **characterised in that** it comprises 0.05 to 20 g per 100 ml of fatty matter of vegetable origin and/or 0.05 to 15 g per 100 ml of fatty matter of animal origin.

14. Use of a bacterial strain according to claim 1 for obtaining products inducing a reduction in blood cholesterol level.

15. Use of a lactic ferment according to any one of claims 2 to 5 for obtaining products inducing a reduction in blood cholesterol level.

16. Use of a fermented product according to any one of claims 8 to 13 for obtaining products inducing a reduction in blood cholesterol level.
